# EUROPEAN PATENT SPECIFICATION

(11) **EP 0 769 301 B1**
(45) Date of publication and mention of the grant of the patent: **31.07.2002**
(21) Application number: 95307497.8
(22) Date of filing: 20.10.1995
(51) Int. Cl.: A61M 5/20, A61D 7/00

(54) **Device for injecting a substance into the body of an animal**
Vorrichtung zum Injizieren einer Substanz in den Körper eines Tieres
Dispositif d'injection d'une substance dans le corps d'un animal

(43) Date of publication of application: 23.04.1997
(73) Proprietor: Genesis Manufacturing Limited, Corsham, Wiltshire (GB)
(72) Inventor: Alexander, Nicholas John, Bacton, Norfolk NR12 0JW (GB); Turley, Roger, Sudbury, Suffolk (GB)
(74) Representative: Carter, Gerald

(56) References cited:
- EP-A- 0 518 416
- EP-A- 0 577 448
- FR-A- 2 477 007
- GB-A- 2 132 488
- GB-A- 2 132 896
- GB-A- 2 162 427
- GB-A- 2 288 539
- US-A- 3 809 083

## Description

The invention relates to a device for injecting a substance into the body of an animal. The device is particularly suitable for use in the case where the animals to be injected are comparatively free to move around or are free range.

Many existing devices are available for injecting livestock. For example, one common form of device is a hand-held injection gun which has a projecting needle which must be introduced into the body of the animal before a trigger mechanism is squeezed to operate a syringe to deliver the injectate along the hollow needle and into the animal. While such injection guns are effective where the animal is closely restrained and comparatively still, they may be difficult to operate with comparatively unrestrained animals in view of the necessity of synchronising the operation of the trigger with the insertion of the needle into the animal.

In order to overcome this problem devices have been proposed which are designed to be simply slapped or prodded against the body of an animal, the impact of the device against the animal being arranged automatically to project a hollow needle through the animal's skin and to discharge a measured dose of injectate through the needle. A device of this general type is disclosed in British Patent Specification No. 2257366 where the momentum of a moving weight is employed to propel injectate into the animal. The present invention sets out to provide an improved form of automatic injection device.

EP 0577448, GB 2132896 and GB 2162427 all disclose slap injectors including a syringe device mounted upon a handle and an actuator arrangement designed to cause actuation of the syringe device automatically upon insertion of the needle of the syringe device into the body of an animal. The device of GB 2162427 includes a pistol-grip type handle. In all three cases, the device is intended to be thrust towards the animal to be injected, in use.

According to the invention there is provided a device for injecting a substance into the body of an animal, comprising: an elongate handle on one end of which is mounted a body part including a syringe device having a hollow needle which projects from the body part; operating means, under the control of a trigger device; and for effecting discharge operation of the syringe device, actuating means mounted on the body part for movement relatively to the body part and said needle whereby, in use, the actuating means engages the body of the animal being injected and is displaced relatively to the body part of the device as the needle passes into the body of the animal, said displacement of the actuating means relatively to the body part being arranged to actuate said trigger device and thereby effect discharge operation of the syringe device.

The elongate handle extends at substantially 90° to the direction of said needle, whereby the needle is introduced into the body of an animal by swinging the handle transverse to its length to bring the actuating means, and then the needle, into contact with the body of the animal, the operating means for effecting discharge operation of the syringe device comprising an operating element which extends longitudinally of the handle and is displaceable by second spring means from a cocked position, in which the second spring means is stressed, to an extended position in which the spring means is released, the operating element being held in the cocked position by the aforementioned trigger device, and being operatively coupled to the syringe device by a mechanical linkage so as to effect discharge operation thereof in moving to said extended position.

Preferably return means are provided to return the actuating means to its initial position relative to the body part, after operation of the syringe device, thereby automatically withdrawing the needle from the body of the animal.

Said return means may comprise first spring means arranged to oppose said initial displacement of the actuating means relatively to the body part so that the spring means returns the actuating means to its initial position after operation of the syringe device.

Releasable latch means may be provided to retain the actuating means temporarily in its displaced position while discharge operation of the syringe device is effected, said latch means being released upon completion of said discharge operation to allow the actuating means to be returned to its initial position.

Said actuating means may include a slap plate which extends transversely to the hollow needle and has an aperture through which the needle projects as the slap plate is displaced relatively to the body part. The apertured slap plate thus serves to guide and support the needle as it is inserted into the animal, thus reducing the risk of the needle being bent.

The outer surface of the slap plate is preferably convexly curved with the needle aperture located at the lowermost point of the curve. This ensures that the full length of the needle passes into the animal even if the device is not applied perfectly squarely to the animal's hide.

The actuating means may also include a peripheral wall which surrounds and shields the needle when the actuating means is in its initial position.

The mechanical linkage is preferably arranged such that the movement of the syringe device is less than that of the operating element, so as to provide a mechanical advantage. For example, said mechanical linkage may include a pivoted lever, one end of which is operatively coupled to the syringe device and the opposite end of which is operatively coupled, e.g. through a pivoted link, to the operating element.

Preferably part of the mechanical linkage bears on a movable part of the syringe device, and is not positively connected thereto, whereby the syringe device may be readily separated from the operating means.

The syringe device may comprise a piston and cylinder assembly, the operating means being arranged to displace the piston in the cylinder, to discharge injectate therefrom, as it moves from its cocked to its extended position. Preferably third spring means are disposed to oppose discharge movement of the piston in the cylinder, whereby the third spring means returns the piston to its initial position as the operating means is moved to its cocked position.

Preferably the injecting device includes a fluid reservoir from which injectate fluid is delivered to the syringe device for discharge.

The following is a detailed description of an embodiment of the invention, by way of example, reference being made to the accompanying drawings in which:
Figure 1 is a diagrammatic cross section through one form of injection device according to the present invention, shown prior to cocking, and
Figures 2 shows the operating mechanism of the device in the cocked position.

The injection device comprises an operating head 10 mounted on one end of an elongate handle 11. The head 10 is generally cylindrical and extends at 90° to the length of the handle 11. For strength and durability, all of the structural components of the device may be formed from metal, but it will be appreciated that, in the case of some components, suitable plastics or other materials might well be used.

The operating head 10 comprises a cylindrical main body 12 formed with a central bore 13 leading from an upper transverse cross passage 14 which communicates with a hollow end portion of the handle 11.

The main body 12 incorporates a syringe device. For this purpose the lower end of the bore 13 is enlarged in diameter and encloses a cylinder 15 which is held in the bore 13 by a nut 16. A tube 17 leads downwardly from the cylinder 15 and is coupled by means of a needle holder 18 to a removable hollow needle 19. A non-return valve 20 is provided in the tube 17.

A plunger 21 is reciprocable in the cylinder 15 and is mounted on the lower end of a tubular piston rod 22. The lower end of the tubular rod 22 communicates with the interior of the cylinder 15 through a bore 23 in the plunger 21, a non-return valve 24 being provided in the bore 23.

The plunger 21 and piston rod 22 are biased upwardly by a helical compression spring 25 which encircles the rod 22 between the upper surface of the cylinder 15 and the under surface of a cap member 26 which is secured to the piston rod 22 adjacent the cross passage 14 in the main body 12. The upper end of the tubular piston rod 22 is connected to a flexible conduit 27 which extends along the handle 11 and communicates with a reservoir 28 of injection fluid which is mounted on the handle 11 by means of a clip 29.

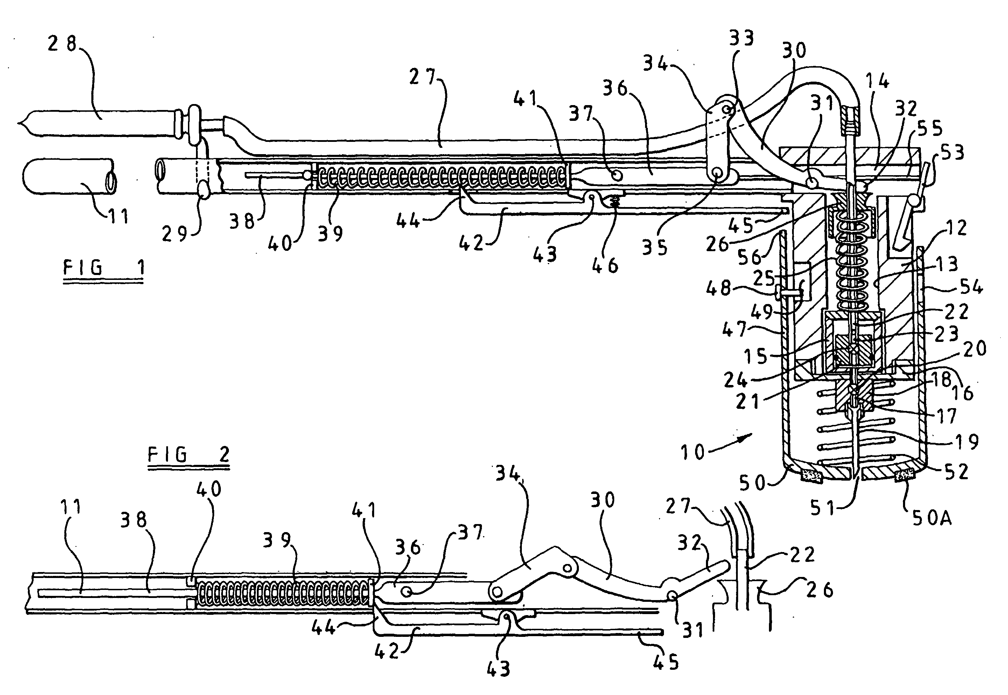

A curved lever 30 is pivotally mounted on the main body 12 by a pivotal mounting 31 and has a pusher part 32 which extends along the passage 14 and bears on the upper surface of the cap member 26. The opposite end of the lever 30 is pivotally connected at 33 to a link 34 which is in turn pivotally connected at 35 to a push rod 36. Cocking bars 37 project outwardly at right angles from opposite sides of the push rod 36 and are slidable along longitudinal slots (not shown) in the sides of the hollow portion of the handle 11. The push rod 36 is connected to a guide rod 38 which extends longitudinally along the interior of the hollow part of the handle 11 and a helical compression spring 39 encircles the guide rod 38 between an annular abutment 40 within the handle 11 and an annular abutment 41 mounted on the end of the push rod 36 at its junction with the guide rod 38. The spring 39 serves to bias the push rod 36 to the right, to the position shown in Figure 1.

A trigger arm 42 is pivotally mounted at 43 on the underside of the handle 11 and has at one extremity a latch part 44 which cooperates with the abutment 41 on the push rod 36. At the opposite extremity the trigger arm has an operating part 45 located adjacent the main body 12 of the injection device. The trigger arm 42 is biased clockwise, as viewed in Figure 1, by a small compression spring 46 located between the trigger and its mounting on the handle 11.

A tubular member 47 is slidable vertically on the body member 12 and has a pin 48 which slides in a vertical guide slot 49 in the body member 12. The lower end of the tubular member 47 normally surrounds the needle 19 and is closed by a circular slap plate 50 formed with a narrow central hole 51 through which the needle 19 may pass. A helical compression spring 52 encircles the needle 19 and needle holder 18 and is disposed between the upper side of the slap plate 50 and the underside of the aforementioned nut 16. The spring 52 serves to bias the hollow member 47 downwardly.

The lower surface of the slap plate 50 is convexly curved, and is formed with an annular groove which receives an annular marking sponge 50A.

A pivoted latch 53 is mounted on the body member 12, above the tubular member 47, and is engageable with an aperture 54 formed in the tubular member 47 adjacent its upper end. The latch 53 also cooperates with an operating rod 55 which extends along the passage 14 from the end of the push rod 36.

The injection device operates as follows:

The device is first cocked by withdrawing the push rod 36 to the left in Figure 1, by pulling on the transverse bars 37. The push rod 36 is withdrawn, compressing the spring 39, until the abutment 41 passes the latch part 44 of the trigger 42 which snaps behind the abutment 41, as shown in Figure 2, under the action of the spring 46. This cocking action serves generally to flatten out the lever 30 and link 34, as shown in Figure 2, swinging the pusher part 32 upwardly. The upward withdrawal of the pusher part 32 allows the piston rod 22 and plunger 21 to be pushed upwardly by the compression spring 25. Due to the presence of the non-return valve 20 in the needle holder this creates a low pressure area in the cylinder 15 causing liquid from the reservoir 28 to be drawn into the cylinder 15 along the flexible conduit 27 and down the hollow piston rod 22. The device is then cocked and primed ready for use.

To inject an animal the user grasps the handle 11 at the end remote from the head 10 and taps the slap plate 50 of the head 10 against an appropriate part of the body of the animal. This tapping action causes the tubular member 47 to slide upwardly on the body part 12, against the action of the spring 52, driving the needle 19 into the body of the animal. During this action the needle 19 remains protected by the lower end of the tubular member 47 and is guided and supported by the hole 51 in the slap plate 50.

As the tubular member 47 reaches the upper limit of its movement on the main body 12 it is engaged by the pivoted latch 53 which engages in the aperture 54 and retains the tubular member 47 in its uppermost position, the operating rod 55 having been withdrawn from the latch 53 by the previous cocking action.

Almost simultaneously with the engagement of the latch 53 the upper edge 56 of the tubular member 47 strikes the operating end 45 of the trigger arm 42 and swings it upwardly. This causes the opposite latch end 44 of the trigger arm 42 to move downwardly out of engagement with the abutment 41 allowing the push rod 36 to be snapped longitudinally to the right, to the position shown in Figure 1, under the action of the compression spring 39. This movement swings the link 34 and lever 30 upwardly to the position shown in Figure 1 and the pusher part 32 of the lever 30 snaps downwardly on the cap member 26 pushing the piston rod 22 and plunger 21 downwardly. The non-return valve 24 in the plunger 21 closes and the non-return valve 20 in the needle holder opens so that the dose of injectate in the cylinder 15 is then delivered downwardly along the needle 19 and into the animal.

As the push rod 36 is snapped to the right to effect the discharge of the injectate, the operating rod 55 connected to it engages the latch 53 and moves it out of engagement with the aperture 54 in the upper part of the tubular member 47. This allows the tubular member 47 to be thrust downwardly by the spring 52. The slap plate 50 bears on the body of the animal causing the needle 19 automatically to be withdrawn from the animal's body. The device is then ready to be re-cocked again ready to inject another animal. It will be noted that fluid is only drawn into the cylinder 15 from the reservoir 28 when the device is cocked, ready to perform an injection, so that there is no injectate remaining in the cylinder when the device is not in use.

Prior to use of the device, the annular sponge 50A on the bottom of the slap plate 50 is soaked with an appropriate marking liquid, so that each time the injection device is used the animal's body is automatically marked with a circle around the injection site, so that it can be seen which animals have been injected. The large area of the slap plate 50 spreads the impact on the animal's body and this, together with the curvature of the undersurface of the slap plate, minimises the pain felt by the animal when struck with the device. The temporary latching of the tubular member 47 in the upper position, by the latch 53, ensures that the needle 19 remains in the animal's body until the injectate has been discharged, whereafter the release of the tubular member 47 drives it downwardly, thus rapidly and automatically withdrawing the needle 19 from the animals body, thus minimising the length of time while the needle is in place, and hence keeps to a minimum the distress and discomfort to the animal.

The mechanical linkage between the push rod 36 and the cap member 26 provides a mechanical advantage so that the plunger 21 of the syringe device can be large enough to expel an adequate dose of injectate with a very small downward movement.

As described, the pusher part 32 simply bears on the upper surface of the cap member 26. It could instead, however, be positively coupled to it, and in this case the cap member 26, piston rod 22 and plunger 21 would be positively raised when the device is cocked, and the spring 25 could be dispensed with. However, the described arrangement is preferred since, in the absence of a positive connection between the syringe assembly and the pusher part 32, the whole syringe assembly may readily and conveniently be withdrawn from the main body 12, by unscrewing the nut 16, for servicing, replacement or repair.

The slap plate 50 serves to shield the needle 19 and to guide and support the needle, thus reducing the chance of bending, as it passes downwardly through the aperture 51. When the needle 19 is withdrawn, the surrounding wall of the tubular member 47 also serves fully to protect the needle against damage.

The arrangement described by way of example employs a mechanical, hand-operated system for operating the syringe assembly. However, the invention includes within its scope arrangements in which the syringe assembly is operated by fluid pressure, for example by compressed air. In this case the mechanical trigger arm 42 would be replaced by a switch to operate an air powered device which, either directly or though a simple linkage, applied the necessary downward movement to the plunger of the syringe assembly. The latch 53 would, in this case, be operated by the member coupled to the syringe assembly, or the linkage driving it, so as to release the latch as the plunger reaches the downward limit of its movement.

## Claims

1. A device for injecting a substance into the body of an animal, including a syringe device (15,21,22,26) having a hollow needle (19), an elongate handle (11) on one end of which is mounted a body part (12) including the syringe device with the hollow needle (19) thereof projecting from the body part (12); operating means (39,30-34), under the control of a trigger device (42), for effecting discharge operation of the syringe device; actuating means (47) mounted on the body part (12) for movement relatively to the body part and said needle whereby, in use, the actuating means (47) engages the body of the animal being injected and is displaced relatively to the body part of the device as the needle passes into the body of the animal, said displacement of the actuating means (47) relatively to the body part (12) being arranged to actuate said trigger device (42) and thereby effect discharge operation of the syringe device (15,21,22,26), **characterised in that** the elongate handle (11) extends at substantially 90° to the direction of the needle (19) to enable the needle (19) to be introduced into the body of an animal by swinging the handle (11) transverse to its length to bring the actuating means (47) and then the needle (19) into contact with the body of the animal, the operating means comprising an operating element (36) extending longitudinally of the handle (11) and moveable by a second spring means (39) from a cocked position in which the second spring means (39) is stressed to an extended position in which the second spring means (39) is released, the operating element (36) being held in its cocked position by the trigger device (42) and being operatively coupled to the syringe device (15,21,22,26) by a mechanical linkage (30-34) to effect discharge operation thereof when the operating element (36) moves towards its extended position.

2. A device according to Claim 1, **characterised in that** return means (52) are provided to return the actuating means (47) to its initial position relative to the body part (12), after operation ofthe syringe device, thereby automatically withdrawing the needle (19) from the body of the animal.

3. A device according to Claim 2, **characterised in that** said return means comprise first spring means (52) arranged to oppose said initial displacement of the actuating means (47) relatively to the body part so that the spring means (52) returns the actuating means to its initial position after operation of the syringe device.

4. A device according to any of Claims 1 to 3, **characterised in that** releasable latch means (53) are provided to retain the actuating means (47) temporarily in its displaced position while discharge operation of the syringe device is effected, said latch means being released upon completion of said discharge operation to allow the actuating means to be returned to its initial position.

5. A device according to any of the preceding claims, **characterised in that** said actuating means (47) include a slap plate (50) which extends transversely to the hollow needle (19) and has an aperture (51) through which the needle projects as the slap plate is displaced relatively to the body part.

6. A device according to Claim 5, **characterised in that** the outer surface of the slap plate (50) is convexly curved with the needle aperture (51) located at the lowermost point of the curve.

7. A device according to any of the preceding claims, **characterised in that** the actuating means (47) include a peripheral wall which surrounds and shields the needle (19) when the actuating means is in its initial position.

8. A device according to any of the preceding claims, **characterised in that** the movement of the syringe device is less than that of the operating element (36), so as to provide a mechanical advantage.

9. A device according to Claim 8, **characterised in that** said mechanical linkage (30-34) includes a pivoted lever (30), one end of which is operatively coupled to the syringe device (15,21,22,26) and the opposite end of which is operatively coupled to the operating element (36).

10. A device according to Claim 8 or Claim 9, **characterised in that** part (32) of the mechanical linkage (30-34) bears on a movable part (26) of the syringe device, and is not positively connected thereto, whereby the syringe device may be readily separated from the operating means.

11. A device according to any of the preceding claims, **characterised in that** the syringe device comprises a piston (21) and cylinder (15) assembly, the operating means being arranged to displace the piston in the cylinder, to discharge injectate therefrom, as it moves from its cocked to its extended position.

12. A device according to any of the preceding claims, **characterised in that** third spring means (25) are disposed to oppose discharge movement of the piston (21) in the cylinder, whereby the third spring means returns the piston to its initial position as the operating means is moved to its cocked position.

13. A device according to any of the preceding claims, **characterised in that** the injecting device includes a fluid reservoir (28) from which injectate fluid is delivered to the syringe device for discharge.

## Patentansprüche

1. Vorrichtung zum Injizieren einer Substanz in den Körper eines Tieres, die umfaßt: eine Injektionsspritzenvorrichtung (15, 21, 22, 26) mit einer hohlen Nadel (19), einem länglichen Griff (11), an dessen einem Ende ein Körperteil (12) montiert ist, das die Injektionsspritzenvorrichtung mit deren hohlen Nadel (19) umfaßt, die aus dem Körperteil (12) herausragt; eine Bedienungseinrichtung (39, 30-34) unter der Kontrolle einer Abzugsvorrichtung (42) für das Bewirken des Entleerungsvorganges der Injektionsspritzenvorrichtung; eine Betätigungseinrichtung (47), die am Körperteil (12) für die Bewegung relativ zum Körperteil und der Nadel montiert ist, wobei die Betätigungseinrichtung (47) beim Gebrauch mit dem Körper des Tieres in Eingriff kommt, das die Injektion erhalten soll, und relativ zum Körperteil der Vorrichtung verschoben wird, während die Nadel in den Körper des Tieres gelangt, wobei die Verschiebung der Betätigungseinrichtung (47) relativ zum Körperteil (12) so eingerichtet wird, daß die Abzugsvorrichtung (42) betätigt und dadurch der Entleerungsvorgang der Injektionsspritzenvorrichtung (15, 21, 22, 26) bewirkt wird; **dadurch gekennzeichnet, daß** sich der längliche Griff (11) unter im wesentlichen 90° zur Richtung der Nadel (19) erstreckt, damit die Nadel (10) in den Körper eines Tieres eingeführt werden kann, indem der Griff (11) quer zu seiner Länge geschwenkt wird, um die Betätigungseinrichtung (47) und danach die Nadel (19) in Berührung mit dem Körper des Tieres zu bringen, wobei die Bedienungseinrichtung ein Bedienungselement (36) aufweist, das sich in Längsrichtung des Griffes (11) erstreckt und mittels einer zweiten Federeinrichtung (39) aus einer gespannten Position, in der die zweite Federeinrichtung (39) gespannt ist, in eine ausgezogene Position beweglich ist, in der die zweite Federeinnichtung (39) freigegeben wird, wobei das Bedienungselement (36) mittels der Abzugsvorrichtung (42) in seiner gespannten Position gehalten wird und funktionell mit der Injektionsspritzenvorrichtung (15, 21, 22, 26) mittels einer mechanischen Verbindung (30-34) gekoppelt ist, um deren Entleerungsvorgang zu bewirken, wenn sich das Bedienungselement (36) in seine ausgezogene Position bewegt.

2. Vorrichtung nach Anspruch 1, **dadurch gekennzeichnet, daß** eine Rückführeinrichtung (52) vorhanden ist, um die Betätigungseinrichtung (47) in ihre Ausgangsposition relativ zum Körperteil (12) nach der Betätigung der Injektionsspritzenvorrichtung zurückzuführen, wodurch die Nadel (19) automatisch aus dem Körper des Tieres zurückgezogen wird.

3. Vorrichtung nach Anspruch 2, **dadurch gekennzeichnet, daß** die Rückführeinrichtung eine erste Federeinrichtung (52) aufweist, die so angeordnet sit, daß sie der anfänglichen Verschiebung der Betätigungseinrichtung (47) relativ zum Körperteil entgegenwirkt, so daß die Federeinrichtung (52) die Betätigungseinrichtung in ihre Ausgangsposition nach der Betätigung der Injektionsspritzenvorrichtung zurückführt.

4. Vorrichtung nach einem der Ansprüche 1 bis 3, **dadurch gekennzeichnet, daß** eine entriegelbare Verriegelungseinrichtung (53) vorhanden ist, um die Betätigungseinrichtung (47) zeitweilig in ihrer verschobenen Position zu halten, während der Entleerungsvorgang der Injektionsspritzenvorrichtung bewirkt wird, wobei die Verriegelungseinrichtung (53) bei Abschluß des Entleerungsvorganges entriegelt wird, damit die Betätigungseinrichtung in ihre Ausgangsposition zurückgeführt werden kann.

5. Vorrichtung nach einem der vorhergehenden Ansprüche, **dadurch gekennzeichnet, daß** die Betätigungseinrichtung (47) eine Anschlagplatte (50) umfaßt, die sich quer zur hohlen Nadel (19) erstreckt und eine Öffnung (51) aufweist, durch die die Nadel herausragt, während die Anschlagplatte relativ zum Körperteil verschoben wird.

6. Vorrichtung nach Anspruch 5, **dadurch gekennzeichnet, daß** die Außenfläche der Anschlagplatte (50) konvex gebogen ist, wobei sich die Nadelöffnung (51) im untersten Punkt der Biegung befindet.

7. Vorrichtung nach einem der vorhergehenden Ansprüche, **dadurch gekennzeichnet, daß** die Betätigungseinrichtung (47) eine periphere Wand umfaßt, die die Nadel (19) umgibt und schützt, wenn sich die Betätigungseinrichtung in ihrer Ausgangsposition befindet.

8. Vorrichtung nach einem der vorhergehenden Ansprüche, **dadurch gekennzeichnet, daß** die Bewegung der Injektionsspritzenvorrichtung geringer ist als die des Bedienungselementes (36), so daß eine Hebelübersetzung bewirkt wird.

9. Vorrichtung nach Anspruch 8, **dadurch gekennzeichnet, daß** die mechanische Verbindung (30-34) einen drehbar gelagerten Hebel (30) umfaßt, von dem ein Ende funktionell mit der Injektionsspritzenvorrichtung (15, 21, 22, 26) gekoppelt ist, und von dem das entgegengesetzte Ende funktionell mit dem Bedienungselement (36) gekoppelt ist.

10. Vorrichtung nach Anspruch 8 oder Anspruch 9, **dadurch gekennzeichnet, daß** das Teil (32) der mechanischen Verbindung (30-34) auf ein bewegliches Teil (26) der Injektioasspritzenvorrichtung einwirkt und nicht zwangläufig damit verbunden ist, wodurch die Injektionsspritzenvorrichtung leicht von der Bedienungseinrichtung getrennt werden kann.

11. Vorrichtung nach einem der vorhergehenden Ansprüche, **dadurch gekennzeichnet, daß** die Injektionsspritzenvorrichtung eine Kolben(21)- und Zylinder(15)baugruppe aufweist, wobei die Bedienungseinrichtung so angeordnet ist, daß der Kolben im Zylinder verschoben wird, um das Injektionsmittel daraus zu entleeren, während sie sich aus ihrer gespannten in ihre ausgezogene Position bewegt.

12. Vorrichtung nach einem der vorhergehenden Ansprüche, **dadurch gekennzeichnet, daß** eine dritte Federeinrichtung (25) angeordnet ist, um der Entleerungsbewegung des Kolbens (21) im Zylinder entgegenzuwirken, wodurch die dritte Federeinrichtung den Kolben in seine Ausgangsposition zurückführt, während die Bedienungseinrichtung in ihre gespannte Position bewegt wird.

13. Vorrichtung nach einem der vorhergehenden Ansprüche, **dadurch gekennzeichnet, daß** die Injektionsvorrichtung einen Fluidbehälter (28) umfaßt, aus dem das Injektionsmittelfluid der Injektionsspritzenvorrichtung zum Entleeren zugeführt wird.

## Revendications

1. Dispositif d'injection d'une substance dans le corps d'un animal, comprenant un dispositif à seringue (15, 21, 22, 26) comportant une aiguille creuse (19), un manche allongé (11) sur une extrémité duquel est montée une partie de corps (12) comprenant le dispositif à seringue dont l'aiguille creuse (19) fait saillie de la partie de corps (12); des moyens de commande (39, 30 à 34), sous la commande d'un dispositif à gâchette (42), pour effectuer une opération de décharge du dispositif à seringue; un moyen d'actionnement (47) monté sur la partie de corps (12) de façon à se déplacer par rapport à la partie de corps et à ladite aiguille, ce par quoi, en utilisation, le moyen d'actionnement (47) coopère avec le corps de l'animal dans lequel s'effectue l'injection et est déplacé par rapport à la partie de corps du dispositif pendant que l'aiguille passe dans le corps de l'animal, ledit déplacement du moyen d'actionnement (47) par rapport à la partie de corps (12) étant destiné à actionner ledit dispositif à gâchette (42) et à effectuer ainsi l'opération de décharge du dispositif à seringue (15, 21, 22, 26), **caractérisé en ce que** le manche allongé (11) s'étend à pratiquement 90° par rapport à la direction de l'aiguille (19) pour permettre l'introduction de l'aiguille (19) dans le corps d'un animal en basculant le manche (11) transversalement à sa longueur afin d'amener le moyen d'actionnement (47) et ensuite l'aiguille (19) en contact avec le corps de l'animal, les moyens de commande comprenant un élément de commande (36) s'étendant longitudinalement au manche (11) et pouvant être déplacé par un deuxième moyen à ressort (39) d'une position armée dans laquelle le deuxième moyen à ressort (39) est contraint à une position détendue dans laquelle le deuxième moyen à ressort (39) est relâché, l'élément de commande (36) étant maintenu dans sa position armée par le dispositif à gâchette (42) et étant couplé fonctionnellement au dispositif à seringue (15, 21, 22, 26) par une liaison mécanique (30 à 34) pour effectuer son opération de décharge lorsque l'élément de commande (36) se déplace vers sa position détendue.

2. Dispositif selon la revendication 1, **caractérisé en ce qu'**un moyen de rappel (52) est prévu pour ramener le moyen d'actionnement (47) à sa position initiale par rapport à la partie de corps (12), après l'opération du dispositif à seringue, rétractant ainsi automatiquement l'aiguille (19) du corps de l'animal.

3. Dispositif selon la revendication 2, **caractérisé en ce que** ledit moyen de rappel comprend un premier moyen à ressort (52) configuré de façon à s'opposer audit déplacement initial du moyen d'actionnement (47) par rapport à la partie de corps de sorte que le moyen à ressort (52) ramène le moyen d'actionnement à sa position initiale après l'opération du dispositif à seringue.

4. Dispositif selon l'une quelconque des revendications 1 à 3, **caractérisé en ce qu'**un moyen de verrouillage libérable (53) est prévu pour retenir temporairement le moyen d'actionnement (47) dans sa position déplacée pendant que l'opération de décharge du dispositif à seringue est effectuée, ledit moyen de verrouillage étant libéré à la fin de ladite opération de décharge pour permettre le retour du moyen d'actionnement à sa position initiale.

5. Dispositif selon l'une quelconque des revendications précédentes, **caractérisé en ce que** ledit moyen d'actionnement (47) comprend une plaque d'appui à coup frappé (50) s'étendant transversalement à l'aiguille creuse (19) et comportant une ouverture (51) à travers laquelle l'aiguille fait saillie pendant le déplacement de la plaque d'appui à coup frappé par rapport à la partie de corps.

6. Dispositif selon la revendication 5, **caractérisé en ce que** la surface extérieure de la plaque d'appui à coupe frappé (50) est recourbée de manière convexe, l'ouverture d'aiguille (51) étant située au point le plus bas de la courbure.

7. Dispositif selon l'une quelconque des revendications précédentes, **caractérisé en ce que** le moyen d'actionnement (47) comprend une paroi périphérique entourant et protégeant l'aiguille (19) lorsque le moyen d'actionnement est dans sa position initiale.

8. Dispositif selon l'une quelconque des revendications précédentes, caractétisé en ce que déplacement du dispositif à seringue est inférieur à celui de l'élément de commande (36) de façon à créer un gain mécanique.

9. Dispositif selon la revendication 8, **caractérisé en ce que** ladite liaison mécanique (30 à 34) comprend un levier pivotant (30) dont une extrémité est couplée fonctionnellement au dispositif à seringue (15, 21, 22, 26) et dont l'autre extrémité est couplée fonctionnellement à l'élément de commande (36).

10. Dispositif selon la revendication 8 ou la revendication 9, **caractérisé en ce que** la partie (32) de la liaison mécanique (30 à 34) s'appuie sur une partie mobile (26) du dispositif à seringue et n'est pas raccordée de façon verrouillée à celui-ci, ce par quoi le dispositif à seringue peut être séparé facilement des moyens de commande.

11. Dispositif selon l'une quelconque des revendications précédentes, **caractérisé en ce que** le dispositif à seringue comprend un ensemble à piston (21) et à cylindre (15), les moyens de commande étant configurés pour déplacer le piston dans le cylindre afin d'en décharger la substance d'injection pendant qu'ils se déplacent de leur position armée à leur position détendue.

12. Dispositif selon l'une quelconque des revendications précédentes, **caractérisé en ce qu'**un troisième moyen à ressort (25) est disposé de façon à s'opposer au mouvement de décharge du piston (21) dans le cylindre, ce par quoi le troisième moyen à ressort ramène le piston à sa position initiale pendant le déplacement des moyens de commande à leur position armée.

13. Dispositif selon l'une quelconque des revendications précédentes, **caractérisé en que** le dispositif d'injection comprend un réservoir de fluide (28) duquel le fluide d'injection est délivré au dispositif à seringue pour être déchargé.
